# EUROPEAN PATENT SPECIFICATION

(11) **EP 1 433 484 B1**
(45) Date of publication and mention of the grant of the patent: **11.04.2007**
(21) Application number: 02029016.9
(22) Date of filing: 27.12.2002
(51) Int. Cl.: A61K 38/20, A61P 17/02, A61P 35/00

(54) **Use of interleukin-18 for treating skin disorders associated with UV radiation**
Verwendung von Interleukin-18 zur Behandlung von UV-assoziierten Hautkrankheiten
Utilisation de l'interleukine-18 dans le traitement des maladies de la peau causées par les radiations UV

(43) Date of publication of application: 30.06.2004
(73) Proprietor: Universitätsklinikum Münster, 48149 Münster (DE)
(72) Inventor: Schwarz, Thomas, Prof. Dr., 48149 Münster (DE); Schwarz, Agatha, Dr., 48149 Münster (DE)
(74) Representative: Krauss, Jan

(56) References cited:
- KISHIDA T. ET AL.: "In vivo electroporation-mediated transfer of interleukin-12 and interleukin-18 genes induces significant antitumor effects against melanoma in mice." GENE THERAPY, vol. 8, no. 16, August 2001 (2001-08), pages 1234-1240, XP009010524
- NAGAI H. ET AL.: "Gene transfer of secreted-type modified IL-18 gene to B16F10 melanoma cells suppresses in vivo tumor growth through inhibition of tumor vessel formation." J. INVEST. DERMATOL., vol. 119, no. 3, September 2002 (2002-09), pages 541-548, XP001148026
- KANG JAE SEUNG ET AL.: "Enhanced IL-18 production by UV-B irradiation requires ROI and AP-1 signaling in human keratinocyte cell line HACAT." FASEB J., vol. 16, no. 4, March 2002 (2002-03), XP009010545
- SCHWARZ A. ET AL.: "Interleukin-12 suppreses ultraviolet radiation-induced apoptosis by inducing DNA repair." NATURE CELL BIOLOGY, vol. 4, January 2002 (2002-01), pages 26-31, XP002241642

## Description

The present invention relates to uses of interleukin-18.

The incidence of disorders of the skin due to environmental influences seems to increase drastically in recent years. Apart from exposure to an ever increasing number and variety of chemicals from various sources, including exhaust fumes from the burning of fossil fuels or from the manufacture of plastic substances, also external radiation plays an increasing part in the genesis of skin disorders. In this context, UV-radiation from both natural origin (i.e. solar radiation) as well as from artificial sources is attributed a leading role in the etiology of skin diseases and disorders. There is a dramatic increase of incidence in both melanoma and non-melanoma skin cancer in Europe, the United States and Australia. The increased influx of UVB radiation due to the decrease of the ozone layer may contribute to this phenomenon to some extent, the major reason for the increase in UV-induced skin cancer is certainly the behaviour of the population. Because of a constant change mostly in the leisure activities the cumulative life time UV dose of the average population has increased over the last decades (Armstrong and Kricker, 2001, J Photochem Photobiol B.;63(1-3):8-18).

In fact, solar ultraviolet radiation is one of the most important environmental influences with respect to the skin of terrestrial life forms. This is particularly true for the medium range of UV-radiation (290-320 nm), because it is this range that is attributed a causative role not only for sunburn, but also for the massive increase in the incidence of skin cancer described above. From a mechanistic point of view the harmful effect of UV-radiation on skin cells is believed to be caused by the massive damage it induces in the DNA of skin cells that have been exposed to such radiation. DNA absorbs UV-light in the UVB-region (290-320 nm) and the UVC-region (200-290 nm). Absorption of UV-radiation is often accompanied by lesions in the DNA-molecules affected. UV-radiation in the range 290-320 nm primarily induces two kinds of photolesions, namely cyclobutane-pyrimidine dimers (CPD) and <6-4> photoproducts. The vast majority of these photoproducts are removed in normal eukaryotic cells by a mechanism, commonly referred to as "nucleotide excision repair" (NER) which is of utmost importance because those DNA-lesions that remain may give arise to malignant aberrations of the cells affected. (de Laat et al., 1999, Genes Dev. 13:768-785). The importance of the NER-mechanism is demonstrated impressively by a genetic disorder characterized by the absence of a functional NER-system, Xeroderma pigmentosum (XP). Accordingly, patients that suffer from Xeroderma pigmentosum exhibit a dramatically increased rate of skin cancer (Kraemer et al., 1994, Proc. Natl. Acad. Sci. USA, 94:1-14, 1997). The eukaryotic NER-system has its prokaryotic and viral counterpart in specific enzymes designed to remove UV-induced DNA-damage. For example an acceleration of DNA-repair can also be achieved by application of exogenous DNA-repair enzymes that remove the same biological UV-effects. The bacterial DNA-repair-enzyme T4N5-endonuclease has been shown to successfully reverse various UV-mediated phenomena including immunosuppression, release of the immunosuppressive cytokine interleukin-10 and the inflammatory mediator tumor necrosis factor α (TNFα), when encapsulated into liposomes. (Wolf et al, J. Invest. Dermatol., 114:149-156, 2000; Kibitel et al., Photochem. Photobiol. 67:541-546, 1998). Similar observations have been made with another repair enzyme, also of bacterial origin, photolyase, which reverses cyclobutane-pyrimidine dimers specifically upon illumination with photoreactivating light, a repair process, also referred to as "photoreactivation" (Stege et al., 2000, Proc. Natl. Acad. Sci. USA, 97:1790-1795). The introduction of these repair enzymes into cells is performed by their incorporation into liposomes and subsequent application of these liposomes onto UV-irradiated cells in culture or onto UV-irradiated skin. It could be shown that that the local application of these repair enzymes significantly reduces the incidence of skin tumors in chronically UV-irradiated mice (Yarosh et al., 1992, Cancer Res. 52,4227-4231). Furthermore, the topical application of T4N5 endonuclease significantly reduces the risk of patients having xeroderma pigmentosum for developing actinic ceratoses (Yarosh et al., 2001, Lancet, 357:926-929). However, these methods have not found their way into the normal treatment of sunburn and skin damage. Conventional protection against sun by using topically applied UV-filters is still undisputedly the most efficient way of preventing UV-induced damage to skin. Conventional UV-filters, however, often carry unwanted side effects, in that they either may cause allergic reactions, if they are UV-absorbing aromatic ring systems, or they do not provide efficient protection by merely mechanical blocking of UV radiation (e.g. metal oxide pigments) and, furthermore, cause soiling of the worn garments.

UV-induced cyclobutane-pyrimidine dimers can activate the tumor suppressor gene p53 which plays a crucial role in the formation of apoptotic keratinocytes that appear in vivo as sunburn cells within the epidermis (Murphy et al., 2001, Exp. Dermatol., 10:155-160). Mice that are deficient in a functional p53 developed significantly fewer sunburn cells upon exposure to UV-irradiation than UV-irradiated wildtype mice (Ziegler et al., 1994, Nature, 372:773-776). Irradiation with UV results in a p53-dependent cell cycle arrest during the G1-phase in order to allow DNA-repair prior to DNA-synthesis. Based on these findings, sunburn cells were assumed to be keratinocytes which have failed to efficiently repair damaged DNA, consequently undergoing apoptosis and thereby escaping the risk of becoming malignant. Accordingly the formation of sunburn cells was considered to be a p53 control scavenging phenomenon which protects individuals from developing UV-induced skin cancer (Brash et al., 1996, J. Invest. Dermatol. Symposium Proceedings, 1:136-142) with regard to the tumor suppressing properties of p53, keratinocytes mutated therein should be susceptible to the tumor promoting effects of UV, whereas other cells carrying wildtype p53 should be eliminated by apoptosis when loaded with a certain amount of DNA damage (Brash et al., ibid.). Since UV preferably mutates p53 (Brash et al., 1991, Proc. Natl. Acad. Sci. USA, 88:10124-10128), it may exert a selective pressure for the mutated, damage resistant keratinocytes, thereby allowing these cells to clonally expand and to form an actinic keratosis, the pre-stage of skin cancer (Ziegler et al., ibid.). Therefore it has been proposed that the severity of DNA-damage is a major mediator of apoptosis and therefore correlates with the number of sunburn cells (Ziegler et al., ibid.). An increase in DNA-repair should also result in a reduction of the apoptotic response of keratinocytes to UV-exposure. Evidence for this assumption was provided when epithelial cells (HeLa) were subjected to enhanced DNA-repair following UV-irradiation. An acceleration of DNA-repair was achieved by photo-reactivation via addition of the DNA-repair enzyme photolyase encapsulated into liposomes. A photoreactivation resulted in a significantly, although not completely, reduced apoptosis rate upon UV exposure (Kulms et al., 1999, Proc. Natl. Acad. Sci. USA, 96:7974-7979). Similar results had been obtained in vivo by demonstrating that a topical application of the repair enzyme T4N5 via liposome delivery reduced the number of sunburn cells both in mice and humans (Wolf et al., 2000, IBID.; Stege et al., 2000, IBID). Taking together, these and the above mentioned data clearly indicate that nuclear DNA damage plays an important role in mediating the biologic effects of UV.

A recent study (Schwarz et al., 2002, Nature Cell Biology, 4:26-31) has shown that interleukin-12 suppresses UV-radiation-induced apoptosis by inducing DNA-repair through the cell's own repair systems, in particular the nucleotide-excision repair system (NER). IL-12 is an immunostimulatory cytokine and has a wide battery of effects. Interleukin-12 is secreted by peripheral lymphocytes after induction. It is produced mainly by B-cells and to lesser extent by T-cells. In terms of its structure, interleukin-12 is a heterodimeric 70 kDa protein having a 35 kDa and a 40 kDa subunit (IL-12A = 35 kDa, 197 amino acids; IL-12 B = 40 kDa, 306 amino acids). The heterodimer is formed by disulfide bonds, formation of which is essential for the biological activity. Both subunits and their interplay seem to be essential for a correct functioning of the molecules, since a 40 kDa subunit-homodimer exhibits antagonistic functions (German et al., 1995, Immunol. Today; 16(10) :500-501)

In terms of its activities, IL-12 is the classical type of an immunostimulatory cytokine, as already mentioned above, and seems to be involved in a wide range of reactions following a primary immune response. IL-12 stimulates the proliferation of human lymphoblasts following a cell activation by phytohemagglutinin. It activates NK-cells which are positive for CD56, and furthermore, it synergizes with anti-CD-3 antibodies with allogeneic stimulation in mixed lymphocyte cultures in inducing T-cell proliferation. Furthermore IL-12 has been shown to induce the synthesis of IFN-γ and interleukin-2 in peripheral lymphocytes of T-helper cells type 1 (Th1). The effects of IL-12 on natural killer cells are mediated by TNF-α, based on the finding that an antibody directed against TNF-α can suppress the effects of IL-12 on natural killer cells. IL-12 and TNF- α are co-stimulators for IFN-γ production with IL-12 maximizing the IFN-γ response. Furthermore, IL-12 functions together with IL-2 in the promotion of the proliferation of mononuclear cells in peripheral blood and in the promotion of the generation of lymphokine-activated killer cells (LAK cells). Picomolar concentrations of IL-12 are as effective as nanomolar concentrations of IL-2 in increasing the cytolytic activity of natural killer cells that have been expanded in vivo by IL-2. Furthermore IL-12 has been implied in the pathogenesis of auto-immune diseases such as multiple sclerosis, contact sensitization and eczema (Riemann et al., 1996, J. Immunol. 156(5) :1799-1803; Leonard et al., 1997, Crit Rev. Immunol. 17(5-6) :545-553). Since IL-12 allows to reduce the doses of IL-2 that are required for the generation of LAK-cells it has been attributed a potential role for adoptive immunotherapy wherein IL-2 is normally used since there are serious adverse side effects associated with interleukin-2, and therefore much hope resides on the co-application of IL-2 with IL-12. IL-12 has also been shown to inhibit the growth of a variety of experimental tumors in vivo and to have an antiangiogenic effect that is believed to be mediated by IFN-γ.

As can be seen from the above, the wide variety of roles IL-12 may have made the use of IL-12 in medical applications a formidable task, since it can not be clearly predicted what the outcome of such an application will be, given the multifactorial nature of the interactions involved. Even to a lesser extent can the insight and knowledge obtained from experiments with IL-12, be simply transferred to other cytokines. Furthermore, the use of IL-12 is severely limited by its high costs and furthermore by its relatively large size, which, for example, rules out topical applications thereof.

The prevention of an onset of UV-induced damaging of the skin after exposure to sunlight and the prevention of premature aging of the skin remains yet to be solved. Conventional means, such as externally applied sun filters do prevent the damaging of the skin, but are useless once a damage has already occurred. The same holds true for mechanical blockers, in the form of cremes, ointments etc. or textile covers, which again, have no use whatsoever once the damage has occurred. Alternative methods, such as the aforementioned application of IL-12 which might prove to be a promising route to follow, but do not seem feasible because of the high costs of IL-12 and its large molecular size which prevents an efficient uptake into the skin.

Accordingly it has been an object of the present invention to provide for an alternative way of preventing and/or treating UV-induced damage to the skin. It is another object of the present invention to provide a medicament for preventing and/or treating UV-induced damage to the skin that is cheaper and more feasible than other medicaments known in the art.

These objects are solved by the use of interleukin-18 for the manufacture of a medicament for the prevention, reduction and treatment of disorders of the skin associated with damage induced by UV-radiation, wherein the disorder is selected from the group comprising sunburn, inflammation, and premature skin aging.

Preferably, the disorder is a disorder that can be alleviated and/or prevented by induction of the nucleotide excision repair (NER) pathway.

It is preferred that the disorder is associated with apoptosis.

In one embodiment, the UV-radiation covers at least a range of wavelengths from 220 nm to 350 nm, preferably from 250 nm to 330 nm, more preferably, from 290 nm to 320 nm.

In one embodiment, the UV-radiation originates from natural and/or artificial sunlight.

Preferably, the use comprises an application of said medicament to a patient in need thereof, wherein, preferably, the application is systemic and/or topical.

In one embodiment, the application occurs by way of application of a pharmaceutically acceptable carrier and/or by injection, preferably intracutaneous injection of a pharmaceutically acceptable carrier, wherein, preferably, the carrier is selected from the group comprising liposomes, ointments, oils, cremes, emulsions and dispersions.

In one embodiment, the topical application occurs in a dose range of from 1 ng/ml to 1000 ng/ml.

In one embodiment, the systemic application occurs in a dose range of from 0.1 µg/kg bodyweight to 100 µg/kg bodyweight, wherein, preferably, the application occurs once to eight times daily.

In one embodiment, the application occurs before, during and after a patient is exposed to UV-radiation.

Preferably, the patient in need is a mammal, preferably a human being.

In making the present invention, the inventors could show that interleukin-18 is capable of preventing and/or treating UV-induced damage in skin that has been irradiated with UV-light. As a consequence, it seems as if interleukin-18 is able to reduce the apoptosis normally associated with UV-radiation. Furthermore interleukin-18 is also capable of securing a long-term survival of keratinocytes that have been irradiated by UV-light. Furthermore, the skin of mice irradiated by UV exhibited fewer apoptotic keratinocytes (sunburn cells (SCs)) when injected with IL-18 than animals without such injection. Without wishing to be bound by any theory, it is presently believed, that the reduction of UV-induced apoptosis can be attributed to a concomitant induction of the repair system of eukaryotic cells, the NER-system. The suppression of the UV-induced apoptosis is attributed to a pronounced reduction of the UV-induced DNA damage, which could be shown by Southwestem-dot-blots (see below). While the amount of UV-induced DNA-damage in groups of mice treated or non-treated with IL-18, was the same immediately after UV-irradiation, the UV-induced DNA-damage was significantly reduced in the group of mice that had been treated with interleukin-18. Similar results could also be found in vivo by means of immuno-histochemical experiments. UV-induced DNA-damage was the same in IL-18-treated or non-treated mice immediately after a UV-irradiation, whereas the DNA-damage was significantly reduced 18 hours after the irradiation. The fact that such an effect could be obtained in wildtype mice, but not in mice that have no functional NER-system, i.e. mice having a deficient Xpa-gene, shows the involvement of the NER-repair system. This furthermore shows that interleukin-18 is capable of preventing UV-induced apoptosis by inducing the NER-system and thereby reducing the DNA-damage caused by the UV-irradiation. Because of such a reduction of DNA-damage, IL-18 has a protective effect against skin cancer and premature skin aging. This is all the more surprising since such an effect had only been observed for interleukin-12, so far, which, functionally, has not been associated with interleukin-18 at all, or only in fields, clearly not involved with the NER-system.

As used herein, the term nucleotide excision repair pathway (NER) is used interchangeably with the term "nucleotide excision repair system" and "nucleotide excision repair mechanism", and generally refers to the system, as for example, described in de Laat et al., 1999, Genes Develop. 13:768-785.

As used herein, the term "disorder associated with apoptosis" is meant to designate a disorder which is caused by and/or accompanied by apoptosis of cells and/or which manifests itself in the apoptosis of cells.

Reference is now made to the figures, wherein
figure 1 shows the suppression of UV-induced apoptosis by IL-18,
figure 2 shows that interleukin-18 enables the survival of keratinocytes after UV-irradiation,
figure 3 shows a reduction of UV-induced DNA-damage by IL-18, in vitro,
figure 4 shows a reduction of UV-induced DNA-damage by IL-18 in vivo, and
figure 5 shows the absence of reduction of sunburn cells by IL-18 in Xpa-deficient mice.

The invention is now described by the following specific examples, which are not intended to limit but merely to illustrate the invention.

### Example 1

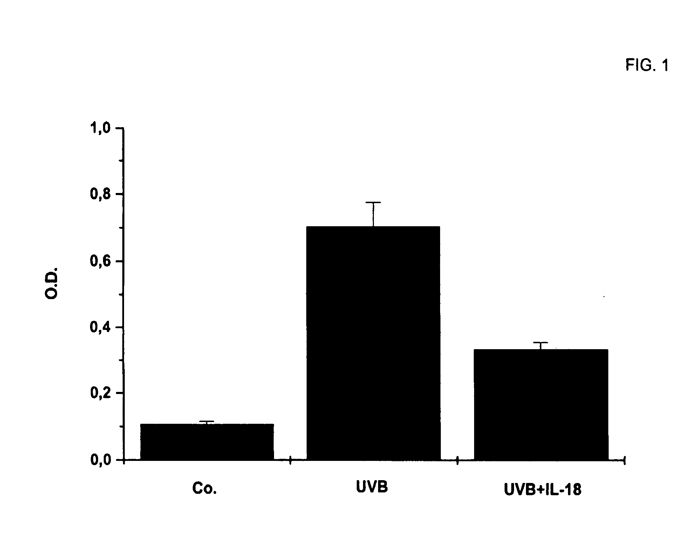

Cell line KB (2x10⁵/ml) was irradiated with 250 J/cm² UVB (290 nm - 320 nm). Four hours before UV-irradiation human recombinant IL-18 (150 ng/ml) was added (UV+IL-18). 16 hours after irradiation the apoptotic cell death was measured by means of an ELISA for apoptosis relying on the principle of DNA-fragmentation. For the detection of DNA fragmentation, a cell death detection ELISA (Boehringer Mannheim) was used. The enrichment of mono- and oligonucleosomes released into the cytoplasm of cells is detected by biotinylated anti-histone- and peroxidase-coupled anti-DNA-antibodies. The rate of apoptosis is reflected in the increase of extinction represented on the Y-axis. Optical density is shown on the y-axis as mean ± SD of triplicates. Student's t-test was used to test the significances of the differences. *p <0.05 (UV+IL-18 vs UV). A result of this experiment can be seen in figure 1, showing that IL-18 suppresses UV-induced apoptosis.

### Example 2

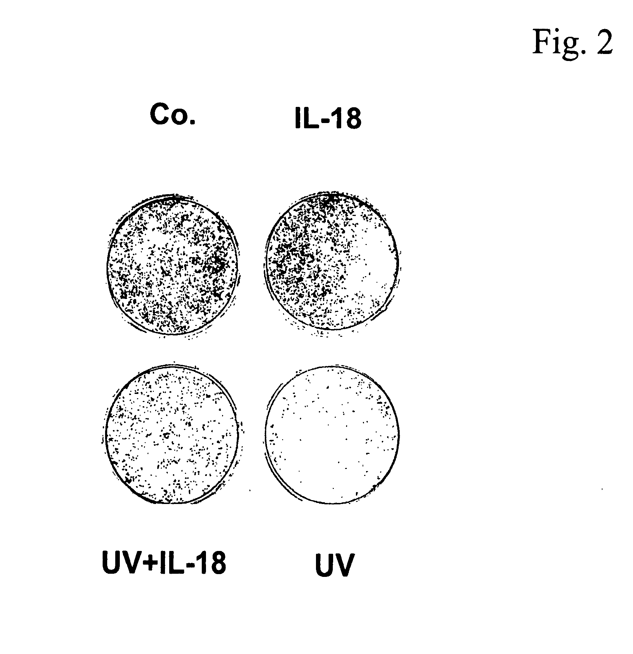

Wildtype human keratinocytes were treated with 150 ng/ml IL-18 or equivalent amounts of diluent, four hours before UV-radiation (250 J/cm²). After UV-irradiation, the cells were plated in medium with and without interleukin-18. 24 hours later medium was replaced by keratinocyte growth medium without IL-18 and cells were cultivated for 21 days. After 21 days colonies were stained with crystal violet, results of which are shown in figure 2. From figure 2 it becomes clear that interleukin-18 enables the survival of keratinocytes after UV-irradiation.

### Example 3

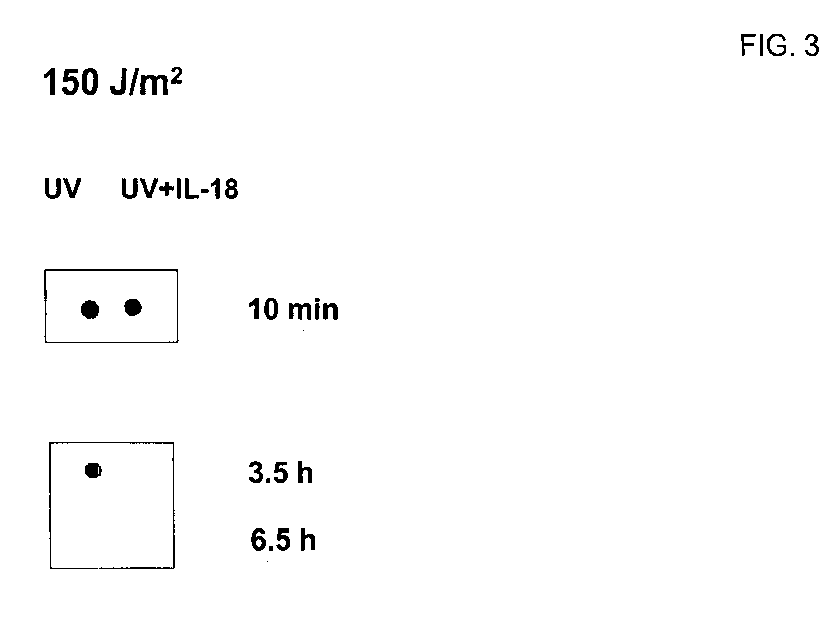

KB-cells were irradiated by UV-light 150 J/cm² (290 - 320 nm) in the presence (UV+IL-18) or absence (UV) of IL-18 (150 ng/ml). 10 minutes, 3.5 and 6.5 hours respectively after UV-irradiation, genomic DNA was extracted and a Southwestem-dot-blot-analysis was performed using an antibody specifically directed UV-induced DNA-damage (cyclobutane-pyrimidine dimers). Genomic DNA was isolated from 1x10⁶ cells according to the DNA extraction protocol from Biozym Diagnostik (Hessisch Oldendorf, FRG). 2 µg genomic DNA were transferred to a positively charged nylon membrane by vacuum-dot-blotting and fixed by baking the membrane for 15 min at 80°C. A monoclonal antibody directed against thymine dimers (Kamiya Biomedical Company, Seattle, WA) was used for Southwestern analysis. Detection was carried out with a horseradish peroxidase-conjugated anti-mouse-antibody. The results of this experiment are shown in figure 3. Figure 3 shows that UV-induced DNA-damage can be reduced by IL-18.

### Example 4

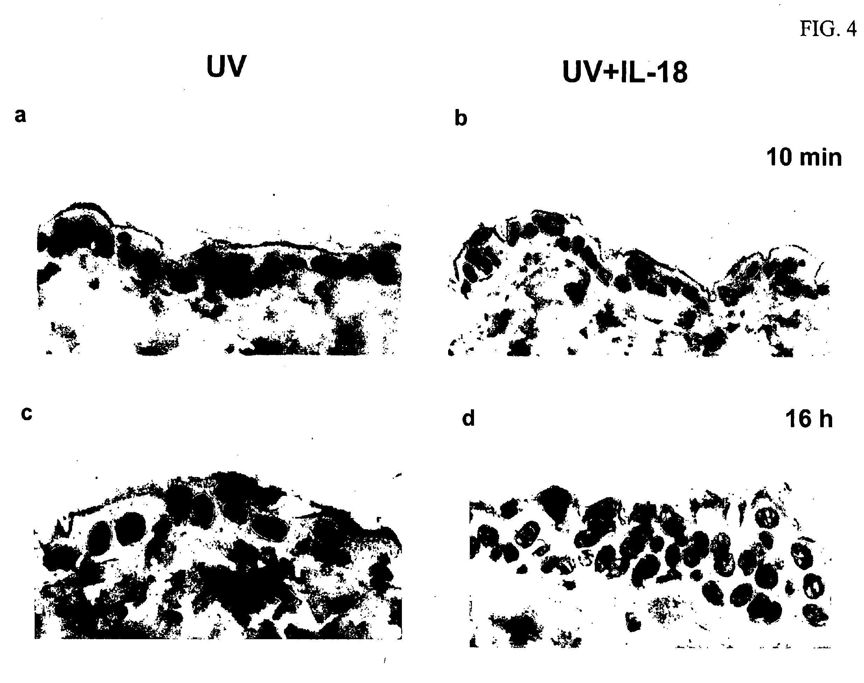

C57BL/6 mice were shaved on their backs and dorsally irradiated with 650 J/cm² UVB. Three hours before UV-radiation, 300 ng IL-18 (b,d) and equal amounts (100 µl) aqueous saline (a,c) were intracutaneously injected. Skin biopsies were taken 10 minutes (a,b) and 16 hours (c,d) post UV-irradiation, and an immunohistochemical staining procedure was performed, using an antibody specifically directed to UV-induced DNA-damage (cyclobutane-pyrimidine dimers). Biopsies were fixed in 7% buffered paraformaldehyde, dehydrated, and embedded in paraffin. After deparaffinisation with xylol and ethanol at decreasing concentrations, sections were immersed in an aqueous solution containing 0.1 M citric acid/0.1 M sodium citrate and subsequently microwave-treated for 15 minutes to unmask antigenic epitopes. Endogenous peroxidase activity was blocked by incubation with 0.1% H₂O₂ and 0.6% sodium acid in PBS for 20 minutes. After rinsing with PBS, unspecific binding sites were blocked with 2% BSA for 30 minutes at room temperature followed by incubation with the primary antibody diluted in 1% BSA overnight. The anti-thymidine-dimer antibody was used at a dilution of 1:2000. Staining was achieved by an indirect immunoperoxidase technique using the following reagents: PBS, peroxidase-conjugated anti-mouse antibody (undiluted, Dako envision, Dako, Hamburg, Germany), 0.01% H₂O₂, 3-amino-9-ethylcarbazole (Sigma Corp., St. Louis, MO). Negative controls comprised omission of the first antibody. Stained sections were evaluated by conventional light microscopy. The results of this experiment are shown in figure 4. As can be seen from figure 4, UV-induced DNA-damage can be reduced in vivo by IL-18.

### Example 5

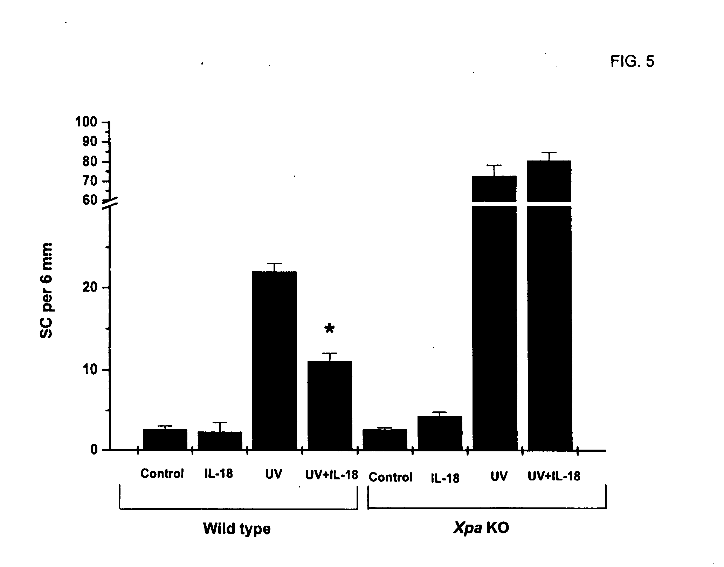

C57BL/6 (Wildtype) and Xpa-deficient mice (Xpa KO) were shaved on their backs and dorsally irradiated by 3000 J/cm² UVB. 3 hours before UV-irradiation, interleukin-18 (300 ng) was intracutaneously injected. 16 hours after UV-irradiation skin biopsies were taken and the number of apoptotic keratinocytes (sunburn cells, SCs) was counted per 6 mm of skin. To evaluate SC-formation, biopsies were taken from the UV-exposed areas of the skin, fixed in formaldehyde, and embedded in paraffin. Tissue sections (5 µm) were stained with hematoxyline and eosin, and the number of SC - defined as apoptotic cells within the epidermis exhibiting a shrunken eosinophilic cytoplasm and a condensed nucleus - was counted throughout the epidermis of sections per sample using a 1 x 1 cm grid inserted in a conventional microscope. SC per 6 millimeter length of epidermis were counted. At least five fields of each sample were evaluated and the number of SC (mean ± SD) calculated. Student's t-test was used to test the significances of the differences. The results of this experiment are shown in figure 5. According to figure 5, in Xpa-deficient mice, UV-induced apoptosis (sunburn cells) cannot be reduced by IL-18, thus implying a role of IL-18 as a direct or indirect inducer of the NER-pathway.

## Claims

1. Use of interleukin-18 for the manufacture of a medicament for the prevention, reduction and treatment of disorders of the skin associated with damage induced by UV-radiation, wherein the disorder is selected from the group comprising sunburn, inflammation, and premature skin aging.

2. Use according to claim 1, wherein the UV-radiation covers at least a range of wavelengths from 220 nm to 350 nm.

3. Use according to any of the foregoing claims, wherein the UV-radiation covers at least a range of wavelengths from 250 nm to 330 nm.

4. Use according to any of the foregoing claims, wherein the UV-radiation covers at least a range of wavelengths from 290 nm to 320 nm.

5. Use according to any of claims 2-4, wherein the UV-radiation originates from natural and/or artificial sunlight.

6. Use according to any of the foregoing claims comprising an application of said medicament to a patient in need thereof.

7. Use according to 6, wherein the application is systemic and/or topical.

8. Use according to any of claims 5-6, wherein the application occurs by way of application of a pharmaceutically acceptable carrier and/or by injection.

9. Use according to claim 8, wherein the application occurs by intracutaneous injection of a pharmaceutically acceptable carrier.

10. Use according to any of claims 8-9, wherein the carrier is selected from the group comprising liposomes, ointments, oils, cremes, emulsions and dispersions.

11. Use according to any of claims 7-10, wherein the topical application occurs in a dose range of from 1 ng/ml to 1000 ng/ml.

12. Use according to any of claims 7-10, wherein the systemic application occurs in a dose range of from 0.1 µg/kg bodyweight to 100 µg/kg bodyweight.

13. Use according to claim 12, wherein the application occurs once to eight times daily.

14. Use according to any of claims 6-13, wherein the application occurs before, during and after a patient is exposed to UV-radiation.

15. Use according to any of claims 6-14, wherein the patient in need is a mammal.

16. Use according to claim 15, wherein the patient in need is a human being.

## Patentansprüche

1. Verwendung von Interleukin-18 zur Herstellung eines Medikaments zur Verhinderung, Verringerung und Behandlung von Hauterkrankungen, die mit einer Schädigung assoziiert sind, welche durch UV-Strahlung induziert wird, wobei die Erkrankung ausgewählt ist aus der Gruppe, umfassend Sonnenbrand, Entzündung und verfrühte Hautalterung.

2. Verwendung nach Anspruch 1, wobei die UV-Strahlung wenigstens einen Wellenlängenbereich von 220 nm bis 350 nm abdeckt.

3. Verwendung nach einem der vorangehenden Ansprüche, wobei die UV-Strahlung wenigstens einen Wellenlängenbereich von 250 nm bis 330 nm abdeckt.

4. Verwendung nach einem der vorangehenden Ansprüche, wobei die UV-Strahlung wenigstens einen Wellenlängenbereich von 290 nm bis 320 nm abdeckt.

5. Verwendung nach einem der Ansprüche 2-4, wobei die UV-Strahlung aus natürlichem und/oder künstlichem Sonnenlicht stammt.

6. Verwendung nach einem der vorangehenden Ansprüche, umfassend eine Verabreichung des Medikaments an einen hierfür bedürftigen Patienten.

7. Verwendung nach Anspruch 6, wobei die Verabreichung systemisch und/oder topisch ist.

8. Verwendung nach einem der Ansprüche 5-6, wobei die Verabreichung mittels Verabreichung eines pharmazeutisch akzeptablen Trägers und/oder mittels Injektion erfolgt.

9. Verwendung nach Anspruch 8, wobei die Verabreichung mittels intrakutaner Injektion eines pharmazeutisch akzeptablen Trägers erfolgt.

10. Verwendung nach einem der Ansprüche 8-9, wobei der Träger ausgewählt ist aus der Gruppe, umfassend Liposomen, Salben, Öle, Cremes, Emulsionen und Dispersionen.

11. Verwendung nach einem der Ansprüche 7-10, wobei die topische Verabreichung in einem Dosisbereich von 1 ng/ml bis 1000 ng/ml erfolgt.

12. Verwendung nach einem der Ansprüche 7-10, wobei die systemische Verabreichung in einem Dosisbereich von 0,1 µg/kg Körpergewicht bis 100 µg/kg Körpergewicht erfolgt.

13. Verwendung nach Anspruch 12, wobei die Verabreichung ein- bis achtmal täglich erfolgt.

14. Verwendung nach einem der Ansprüche 6-13, wobei die Verabreichung erfolgt, bevor, während und nachdem ein Patient UV-Strahlung ausgesetzt wird.

15. Verwendung nach einem der Ansprüche 6-14, wobei der bedürftige Patient ein Säugetier ist.

16. Verwendung nach Anspruch 15, wobei der bedürftige Patient ein Mensch ist.

## Revendications

1. L'utilisation de l'interleukine-18 pour la fabrication d'un médicament pour la prévention, la réduction et le traitement de troubles de la peau liés à un dommage induit par un rayonnement ultraviolet, dans laquelle le trouble est choisi dans le groupe comprenant l'érythème solaire, l'inflammation et le vieillissement prématuré de la peau.

2. L'utilisation selon la revendication 1, dans laquelle le rayonnement ultraviolet couvre au moins un domaine de longueurs d'onde de 220 nm à 350 nm.

3. L'utilisation selon l'une quelconque des revendications précédentes, dans laquelle le rayonnement ultraviolet couvre au moins un domaine de longueurs d'onde de 250 nm à 330 nm.

4. L'utilisation selon l'une quelconque des revendications précédentes, dans laquelle le rayonnement ultraviolet couvre au moins un domaine de longueurs d'onde de 290 nm à 320 nm.

5. L'utilisation selon l'une quelconque des revendications 2 - 4, dans laquelle le rayonnement ultraviolet provient d'une lumière solaire naturelle et/ou artificielle.

6. L'utilisation selon l'une quelconque des revendications précédentes comprenant l'application dudit médicament à un patient le nécessitant.

7. L'utilisation selon la revendication 6, dans laquelle l'application est systémique et/ou topique.

8. L'utilisation selon l'une quelconque des revendications 5 - 6, dans laquelle l'application se fait au moyen de l'application d'un support pharmaceutiquement acceptable et/ou par injection.

9. L'utilisation selon la revendication 8, dans laquelle l'application se fait par injection intracutanée d'un support pharmaceutiquement acceptable.

10. L'utilisation selon l'une quelconque des revendications 8 - 9, dans laquelle le support est choisi dans le groupe comprenant des liposomes, des onguents, des huiles, des crèmes, des émulsions et des dispersions.

11. L'utilisation selon l'une quelconque des revendications 7 - 10, dans laquelle l'application topique se fait à une dose comprise entre 1 ng/ml et 1000 ng/ml.

12. L'utilisation selon l'une quelconque des revendications 7 - 10, dans laquelle l'application systémique se fait à une dose comprise entre 0,1 µg/kg de poids corporel et 100 µg/kg de poids corporel.

13. L'utilisation selon la revendication 12, dans laquelle l'application se fait une à huit fois par jour.

14. L'utilisation selon l'une quelconque des revendications 6 - 13, dans laquelle l'application se fait avant, pendant ou après l'exposition du patient au rayonnnement ultraviolet.

15. L'utilisation selon l'une quelconque des revendications 6 - 14, dans laquelle le patient à traiter est un mammifère.

16. L'utilisation selon la revendication 15, dans laquelle le patient à traiter est un être humain.
